# EUROPÄISCHE PATENTSCHRIFT

(11) **EP 2 164 042 B1**
(45) Veröffentlichungstag und Bekanntmachung des Hinweises auf die Patenterteilung: **08.11.2017**
(21) Anmeldenummer: 08164087.2
(22) Anmeldetag: 10.09.2008
(51) Int. Cl.: G06T 7/00, A61B 90/00

(54) **Verfahren zur Überprüfung der relativen Lage von Knochenstrukturen**
Method for testing the relative position of bone structures
Procédé de vérification de la position relative de structures osseuses

(43) Veröffentlichungstag der Anmeldung: 17.03.2010
(73) Patentinhaber: Brainlab AG, 81829 München (DE)
(72) Erfinder: Mahn, Florian, 83627 Warngau (DE); Kleinszig, Gerhard, 85551 Kirchheim (DE)
(74) Vertreter: Schwabe - Sandmair - Marx

(56) Entgegenhaltungen:
- US-A- 5 291 537
- US-A1- 2005 119 593
- DUMAS R ET AL P ET AL: "Validation of the relative 3D orientation of vertebrae reconstructed by bi-planar radiography" MEDICAL ENGINEERING & PHYSICS ELSEVIER UK, Bd. 26, Nr. 5, Juni 2004 (2004-06), Seiten 415-422, XP002513821 ISSN: 1350-4533
- BENAMEUR S ET AL: "A hierarchical statistical modeling approach for the unsupervised 3-D biplanar reconstruction of the scoliotic spine" IEEE TRANSACTIONS ON BIOMEDICAL ENGINEERING, IEEE SERVICE CENTER, PISCATAWAY, NJ, US, Bd. 52, Nr. 12, 1. Dezember 2005 (2005-12-01), Seiten 2041-2057, XP007902896 ISSN: 0018-9294
- BENAMEUR S ET AL: "3D/2D registration and segmentation of scoliotic vertebrae using statistical models" COMPUTERIZED MEDICAL IMAGING AND GRAPHICS, PERGAMON PRESS, NEW YORK, NY, US, Bd. 27, Nr. 5, 1. September 2003 (2003-09-01), Seiten 321-337, XP002322203 ISSN: 0895-6111
- PETIT Y ET AL: "ESTIMATION OF 3D LOCATION AND ORIENTATION OF HUMAN VERTEBRAL FACET JOINTS FROM STANDING DIGITAL RADIOGRAPHS" MEDICAL AND BIOLOGICAL ENGINEERING AND COMPUTING, SPRINGER, HEILDELBERG, DE, Bd. 36, Nr. 4, 1. Juli 1998 (1998-07-01), Seiten 389-394, XP000755436 ISSN: 0140-0118
- GRUESCU C ET AL.: "Relative rotation angle between spinal vertebrae using complex shape markers" ANNALS OF THE ORADEA UNIVERSITY, FASCICLE OF MANAGEMENT AND TECHOLOGICAL ENGINEERING, [Online] Bd. VII, 30. Mai 2008 (2008-05-30), Seiten 847-852, XP002513820 Oradea, Romania Gefunden im Internet: URL:http://imtuoradea.ro/auo.fmte/MECATRON ICA_files/GRUESCU%20CORINA%201.pdf> [gefunden am 2009-02-03]
- VERDONCK B ET AL: "COMPUTER ASSISTED QUANTITATIVE ANALYSIS OF DEFORMITIES OF THE HUMAN SPINE" MEDICAL IMAGE COMPUTING AND COMPUTER-ASSISTED INTERVENTION.MICCAI. INTERNATIONAL CONFERENCE. PROCEEDINGS, XX, XX, 1. Januar 1998 (1998-01-01), Seiten 822-831, XP000869944
- YAN KANG ET AL: 'A new accurate and precise 3-D segmentation method for skeletal structures in volumetric CT data' IEEE TRANSACTIONS ON MEDICAL IMAGING. Bd. 22, Nr. 5, 01 Mai 2003, US, Seiten 586 - 598, XP055275062 DOI: 10.1109/TMI.2003.812265 ISSN: 0278-0062

## Beschreibung

Die vorliegende Erfindung betrifft ein Verfahren zur Überprüfung der relativen Lage mindestens zweier Knochenstrukturen, insbesondere mindestens zweier Wirbel einer Wirbelsäule, zueinander.

Bei der Durchführung einer Operation ist es oftmals notwendig, die relative Lage von Knochenstrukturen zueinander zu kennen. Knochenstrukturen sind beispielsweise Wirbel einer Wirbelsäule oder Bruchstücke eines Knochens. Bei Knochenbrüchen ist die korrekte Lage der Bruchstücke entscheidend für ein korrektes Zusammenwachsen. Die Lage von Wirbeln einer Wirbelsäule ist wichtig bei der Implantation künstlicher Bandscheiben, einer,Implantation von Cages, der Versteifung zweier Wirbel (Interbody Fusion), beim Einsetzen von künstlichen Facettengelenken oder einer dynamischen Stabilisierung.

Eine Möglichkeit besteht darin, während der Operation wiederholt 3D-Bilder anzufertigen. Dies ist jedoch zeitaufwendig und die dazu notwendige Vorrichtung ist im Operationsumfeld hinderlich. Eine andere Möglichkeit besteht darin, jede Knochenstruktur mit einer Markiervorrichtung zu versehen und die relativen Lagen der markierten Vorrichtungen beispielsweise über eine 3D-Kamera zu detektieren. Dies setzt eine Beschädigung der Knochenstruktur voraus und erfordert eine zusätzliche oder vergrößerte Körperöffnung, durch die eine Markervorrichtung hindurchtritt. Die wissenschaftliche Veröffentlichung von Dumas et al.:"Validation of the relative 3D orientation of vertebrae reconstructed by bi-planar radiography" Medical Engineering & Physics Elsevier UK, Bd. 26, Nr. 5, Juni 2004, Seiten 415-422 beschreibt die Segmentierung und Lagebestimmung einzelner Wirbelkörpern in einem Modell einer Wirbelsäule. Es ist daher die Aufgabe der vorliegenden Erfindung, ein Verfahren zur Überprüfung der relativen Lage mindestens zweier Knochenstrukturen bereitzustellen, das schnell, kostengünstig und für den Patienten möglichst wenig belastend ist.

Gelöst wird diese Aufgabe durch das Verfahren nach Patentanspruch 1. Der Patentanspruch 11 betrifft ein Programm zur Durchführung des vorgenannten Verfahrens und der Patentanspruch 12 betrifft ein Speichermedium, auf dem dieses Programm gespeichert ist. Der Patentanspruch 13 schließlich betrifft eine Vorrichtung, auf der das Programm ausgeführt wird.

Bei dem erfindungsgemäßen Verfahren zur Überprüfung der relativen Lage mindestens zweier Knochenstrukturen (Knochen), insbesondere mindestens zweier Wirbel einer Wirbelsäule, zueinander wird zunächst ein dreidimensionaler Datensatz aufgenommen, der die Knochenstrukturen (Knochen) darstellt. Anschließend wird der dreidimensionale Datensatz in Segmente segmentiert, wobei jedes Segment eine Knochenstruktur (einen Knochen) darstellt und die Segmente im dreidimensionalen Datensatz eine bekannte relative Lage aufweisen. Soweit hierin von Knochenstrukturen die Rede ist, umfasst dies insbesondere den Begriff der im anatomischen Körper vorhandenen (einzelnen) Knochen.

Bei der Segmentierung werden die Einträge des dreidimensionalen Datensatzes, beispielsweise die Voxel eines 3D-Bildes, den Segmenten zugeordnet. Einträge des dreidimensionalen Datensatzes, die nicht einer Knochenstruktur zurechenbar sind (beispielsweise sonstiges Gewebe), werden entweder keinem Segment zugeordnet oder auf die anderen Segmente verteilt. Somit repräsentiert jedes Segment genau eine der Knochenstrukturen (genau einen Knochen). Der dreidimensionale Datensatz enthält bevorzugt die räumliche Dichteverteilung der Knochenstrukturen.

In einem weiteren Schritt wird die relative Lage der Segmente virtuell zu einer virtuellen relativen Lage geändert, sodass die virtuelle relative Lage der Segmente mit der realen relativen Lage der Knochenstrukturen übereinstimmt. Dazu werden die Segmente virtuell gegeneinander verschoben und/oder verdreht. Die virtuelle relative Lage der Segmente weicht dann insbesondere von derjenigen relativen Lage ab, die die Segmente im dreidimensionalen Datensatz einnehmen und entspricht derjenigen relativen Lage, die auch die realen Knochenstrukturen des Patienten zueinander einnehmen. Dies wird auch als Registrierung bezeichnet. Schließlich wird überprüft, ob die virtuelle relative Lage der Segmente mit einer Solllage der Knochenstrukturen übereinstimmt. Dazu wird die Registrierung verwendet. Durch die Registrierung wird die Istlage der realen Knochenstruktur bestimmt und somit eine Möglichkeit geschaffen, eine Abweichung der Istlage von der Solllage zu bestimmen.

Der Begriff "Lage" umfasst in diesem Dokument sowohl die (räumliche) Position als auch eine (räumliche) Orientierung. Eine Lage ist beispielsweise bezogen auf ein Referenzobjekt wie eine Knochenstruktur, eine Markiereinrichtung oder den Operationstisch. Auch bei der Solllagc handelt es sich um eine relative Lage von Strukturen zueinander.

Besonders bevorzugt eignet sich das Verfahren für drei, vier oder mehr Knochenstrukturen bzw. Wirbel. In einem vorteilhaften Anwendungsfall der vorliegenden Erfindung wird durch eine Vielzahl von Segmenten ein Verlauf der Knochenstrukturen beschrieben, beispielsweise der gekrümmte Verlauf von Wirbeln einer Wirbelsäule.

Das Aufnehmen des dreidimensionalen Datensatzes erfolgt bevorzugt präoperativ. Damit ist es möglich, die unter Umständen aufwändige Gewinnung und Segmentierung des dreidimensionalen Datensatzes ohne zeitliche Beschränkungen durchzuführen. Der dreidimensionale Datensatz wird beispielsweise durch eine Computertomographie (CT) oder eine Magnetresonanztomographie (MRT) gewonnen.

Gemäß einer Ausführungsform der Erfindung erfolgt die Änderung der virtuellen relativen Lage der Segmente anhand von CT-Fluoro-Matching. Dabei werden die Lagen der Segmente zueinander virtuell verändert, bis virtuelle Röntgenaufnahmen der entsprechend zueinander angeordneten Segmente mit den realen Röntgenaufnahmen der Knochenstrukturen übereinstimmen. Eine virtuelle Röntgenaufnahme wird aus der Lage der Segmente und der Blickrichtung, aus der die Röntgenaufnahme generiert wird, berechnet.

In einer alternativen Ausgestaltungsform erfolgt die Änderung der virtuellen relativen Lage der Segmente dadurch, dass Landmarken der Knochenstrukturen mittels eines Pointers abgetastet werden und die virtuelle relative Lage der Segmente geändert wird, sodass korrespondierende Landmarken der Segmente mit den abgetasteten Landmarken übereinstimmen. Dies erfordert weniger Rechenleistung, setzt jedoch den Zugang zu den Landmarken der Knochenstrukturen voraus.

Bevorzugt erfolgt die Überprüfung, ob die virtuelle relative Lage der Segmente mit einer Solllage der Knochenstrukturen übereinstimmt, dadurch, dass jedem Segment des dreidimensionalen Datensatzes ein individuelles Bezugssystem zugeordnet wird und die relative Lage der Bezugssysteme mit einer Solllage der Bezugssysteme verglichen wird. Die Solllage der Bezugssysteme korrespondiert zu der Solllage der Knochenstrukturen. Wenn jedem Segment ein Bezugssysteme fest zugeordnet wird, nehmen die Bezugssysteme der Segmente ihre Solllage zueinander ein, wenn auch die Segmente und damit die Knochenstrukturen ihre Solllage zueinander einnehmen. Die Bezugssysteme werden den Segmenten beispielsweise direkt nach der Segmentierung des dreidimensionalen Datensatzes oder direkt vor der Überprüfung, ob die virtuelle relative Lage der Segmente mit einer Solllagc der Knochenstrukturen übereinstimmt, zugeordnet.

In einer Ausgestaltungsform der Erfindung besteht ein Bezugssysteme aus einer Sagittalebene und einer Koronarebene. Handelt es sich bei der Knochenstruktur um einen Wirbel einer Wirbelsäule, ist die Sagittalebene bevorzugt die Mittsagittalebene, die üblicherweise eine horizontale Symmetrieebene des Wirbels darstellt. Die Koronarebene enthält bevorzugt die Endpunkte der Querfortsätze des Wirbels. Der Winkel zwischen den derart definierten Koronarebenen zweier benachbarter Wirbel entspricht direkt dem Lordose- bzw. Kyphosewinkel. Der Winkel zwischen den Mittsagittalebenen benachbarter Wirbel entspricht der Torsion zwischen den beiden Wirbeln. Lordose bzw. Kyphose bezeichnen die Krümmung der Wirbelsäule in der Sagittalebene.

Alternativ handelt es sich bei dem Bezugssystem um ein Koordinatensystem oder eine Menge von Punkten, beispielsweise charakteristischer Punkte wie Landmarken der Knochenstruktur.

Bei Wirbeln einer Wirbelsäule wird die Solllage der Knochenstrukturen beispielsweise durch Festlegen eines Lordosewinkels bzw. Kyphosewinkels definiert.

Bei der Überprüfung, ob die relative Lage der Knochenstrukturen der vorbestimmten relativen Lage der Knochenstrukturen entspricht, ist bevorzugt eine vorgebbare Abweichung zulässig. Dadurch werden die beiden Lagen auch dann als gleich angesehen, wenn sie voneinander abweichen, beispielsweise im Rahmen der Messgenauigkeit oder medizinisch zulässiger Toleranzen.

Optional wird aus den Segmenten und ihrer virtuellen relativen Lage eine Ansicht der Knochenstrukturen in ihrer relativen Lage berechnet und dargestellt. Die Ansicht wird beispielsweise auf einem Monitor wiedergegeben. Damit erhält der Operateur eine Darstellung der aktuellen relativen Lage der Knochenstrukturen.

Die Erfindung betrifft weiterhin ein Programm, das, wenn es in eine Datenverarbeitungseinrichtung geladen wird oder auf einer Datenverarbeitungseinrichtung läuft, die Datenverarbeitungseinrichtung veranlasst, eines der vorstehend beschriebenen Verfahren auszuführen. Das Programm ist beispielsweise auf einem Speichermedium gespeichert oder wird als Information von einer Signalwelle getragen. Eine erfindungsgemäße Vorrichtung weist einen Computer auf, auf dem das vorstehend genannte Programm läuft oder geladen ist.

Die vorliegende Erfindung soll anhand mehrerer Ausführungsbeispiele näher beschrieben werden. Dabei zeigt
- Figur 1: das Grundprinzip des Verfahrens in zweidimensionaler Darstellung,
- Figur 2: ein alternatives Bezugssystem,
- Figur 3: eine grafische Visualisierung eines dreidimensionalen Datensatzes,
- Figuren 4A und 4B: zwei Röntgenaufnahmen,
- Figur 5: eine Vorrichtung zur Durchführung des Verfahrens, und
- Figur 6: ein Flussdiagramm für ein Verfahren zur Überprüfung der relativen Lage von Knochenstrukturen.

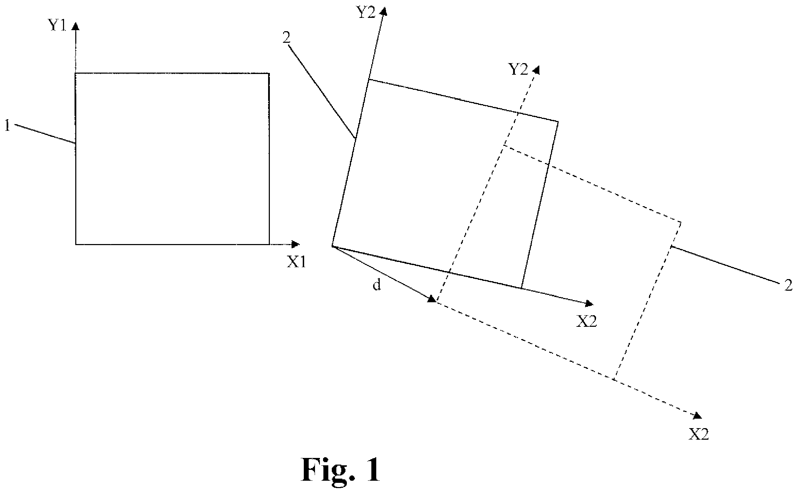

Die Figur 1 zeigt schematisch zwei Knochenstrukturen 1 und 2, wobei zur Erläuterung des Grundprinzips des Verfahrens eine zweidimensionale Darstellung gewählt wurde. Die Knochenstrukturen 1 und 2 sind als Rechtecke dargestellt, wobei die Knochenstruktur 1 als Referenz dient. Die durchgezogene Darstellung der Knochenstruktur 2 zeigt diese in einer ersten Lage, die mittels eines Computertomographen ermittelt wurde. Der Knochenstruktur 1 ist als Bezugssystem ein kartesisches Koordinatensystem mit den Achsen X1 und Y1 zugeordnet, der Knochenstruktur 2 ist als Bezugssystem ein kartesisches Koordinatensystem mit den Achsen X2 und Y2 zugeordnet. Mithilfe des Computertomographen wurde ein dreidimensionaler Datensatz erzeugt, der anschließend in zwei Segmente segmentiert wurde. Das erste Segment stellt die Knochenstruktur 1 dar, das zweite Segment die Knochenstruktur 2. Die Segmente dienen als Muster zum späteren Auffinden der realen relativen Lage der Knochenstrukturcn. Die Lage der beiden Bezugssysteme im dreidimensionalen Datensatz ist bekannt, da die Lage der Segmente im dreidimensionalen Datensatz bekannt ist.

In der Figur 1 gestrichelt dargestellt ist die zu einem späteren Zeitpunkt registrierte Lage der Knochenstruktur 2 in Bezug auf die Knochenstruktur 1. Im Gegensatz zu der durchgezogen dargestellten Lage ist die Knochenstruktur 2 um den Vektor d verschoben und um einen in der Figur 1 nicht dargestellten Winkel α im Uhrzeigersinn verdreht. Zur Bestimmung der Lage der Knochenstrukturen wird die virtuelle relative Lage der Segmente verändert, sodass die virtuelle relative Lage der Segmente mit der realen relativen Lage der Knochenstrukturen übereinstimmt. Dies geschieht beispielsweise durch sukzessives Verändern der relativen virtuellen Lage der Segmente und einen Abgleich dieser Lage mit der realen relativen Lage der Knochenstrukturen anhand von CT-Fluoro-Matching oder dadurch, dass abgetastete Landmarken der Knochenstrukturen mit korrespondierenden Landmarken in den Segmenten in Übereinstimmung gebracht werden.

Das Ergebnis ist eine bekannte Veränderung der relativen Lage der Segmente, also eine Verschiebung und/oder Verdrehung, gegenüber der ursprünglichen relativen Lage im dreidimensionalen Datensatz. Da die Bezugssysteme fest an den Segmenten verankert sind, entspricht diese Veränderung der Veränderung der relativen Lage der Bezugssysteme. Die aktuelle relative Lage der Bezugssysteme zum Zeitpunkt der Registrierung entspricht der ursprünglichen relativen Lage der Bezugssysteme im dreidimensionalen Datensatz überlagert mit der Veränderung der relativen Lage der Bezugssysteme. Zur Überprüfung der relativen Lage der Knochenstrukturen mit einer Solllage der Knochenstrukturen wird die relative Lage der Bezugssysteme zum Zeitpunkt der Registrierung numerisch mit der Solllage der Bezugssysteme verglichen.

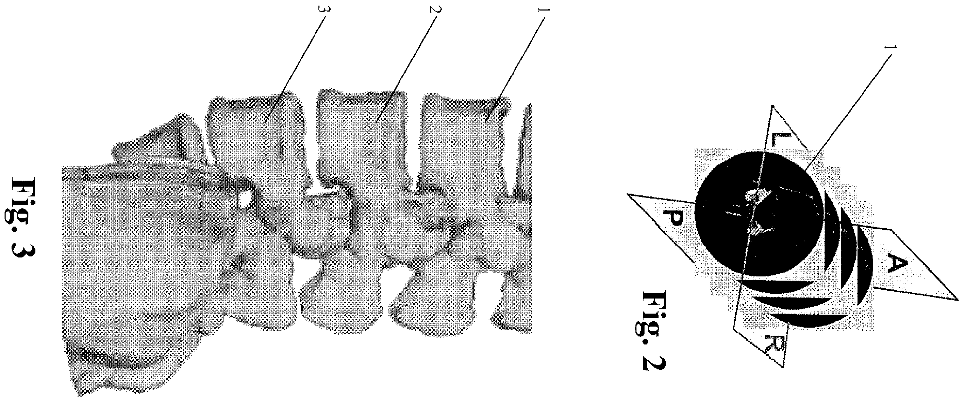

Dargestellt in der Figur 2 ist die Verwendung von Ebenen als Bezugssystem als Alternative zu einem (kartesischen) Koordinatensystem. Bei der Knochenstruktur 1 handelt es sich um den Wirbel einer Wirbelsäule. Als Bezugssystem dienen die Ebenen AP sowie LR. Die Ebene AP ist die Mittsagittalebene, die zentral durch den Dornfortsatz verläuft. Die Ebene LR ist die Koronarebene, in der die Endpunkte der Querfortsätze des Wirbels liegen. Ein derart definiertes Bezugssysteme ist besonders vorteilhaft, da die Ebenen unmittelbar medizinisch relevanten Parametern entsprechen. So entspricht der Winkel zwischen den LR-Ebenen zweier benachbarter Wirbel dem Lordose- bzw. Kyphosewinkel zwischen den Wirbeln.

Die Figur 3 zeigt eine grafische Visualisierung eines dreidimensionalen Datensatzes, der einen Teil einer Wirbelsäule repräsentiert. Der Datensatz bildet eine räumliche Dichteverteilung ab. In dem in Figur 3 dargestellten Bild sind unter anderem die Wirbel 1, 2 und 3 zu erkennen. Der dreidimensionale Datensatz wird in Segmente unterteilt, wobei jedes Segment einen der Wirbel enthält. Nun wird jedem der Segmente ein individuelles Bezugssystem zugeordnet. Dies erfolgt beispielsweise automatisch anhand einer Analyse des Bilddatensatzes oder manuell. Bei einem Bezugssystem handelt es sich, wie vorstehend beschrieben, um ein Koordinatensystem oder eine Menge von Ebenen. Die Lage der Bezugssysteme zueinander repräsentiert die Lage der Wirbel zueinander zum Zeitpunkt der Aufnahme des dreidimensionalen Datensatzes. Die relative Lage der Bezugssysteme ist bekannt, da die relative Lage der Segmente im dreidimensionalen Datensatz bekannt ist.

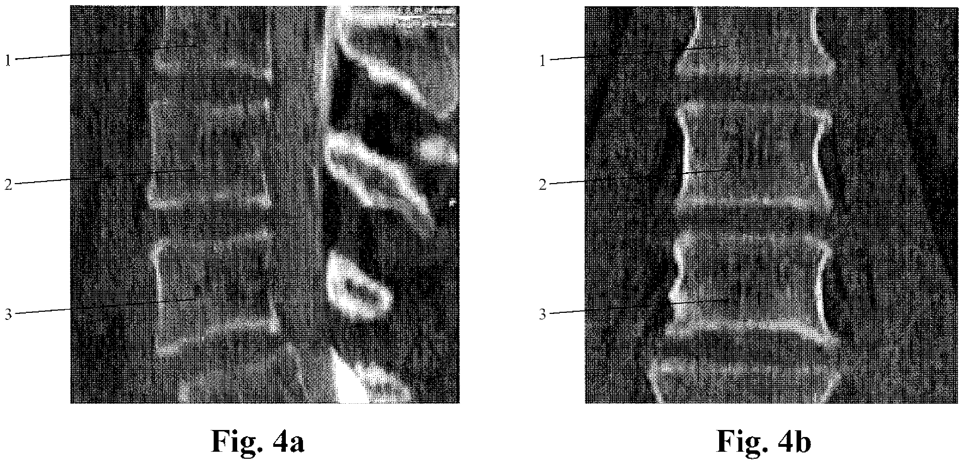

In einem weiteren Schritt wird die tatsächliche Lage der Wirbel 1, 2 und 3 zu einem späteren Zeitpunkt bestimmt, im vorliegenden Beispiel anhand des CT-Fluoro-Matching. Dazu werden zweidimensionale Röntgenbilder aufgenommen, wie sie in den Figuren 4A und 4B dargestellt sind. Die Figur 4A zeigt eine von der Seite aufgenommene Röntgenaufnahme und die Figur 4B eine von vorne aufgenommene Röntgenaufnahme des Teils der Wirbelsäule aus Figur 3.

Aus den beiden Röntgenaufnahmen und dem segmentierten dreidimensionalen Datensatz werden Lageänderungsdaten der Segmente berechnet. Die Segmente des dreidimensionalen Datensatzes werden dabei schrittweise virtuell gegeneinander verschoben und/oder gedreht. Aus der neuen Anordnung der Segmente werden dann in jedem Schritt virtuelle Röntgenbilder berechnet. Stimmen diese virtuellen Röntgenbilder mit den aufgenommenen Röntgenbildern überein, so entspricht die virtuelle Lage der Segmente der tatsächlichen Lage der Wirbel. Die Änderungen der Lagen der Segmente gegenüber ihrer Lage in dem ursprünglichen dreidimensionalen Datensatz werden zu den Lageänderungsdaten zusammengefasst. Die relative Lage der Bezugssysteme zueinander zum Zeitpunkt der Aufnahme der Röntgenbilder, also zum Zeitpunkt der Registrierung, entspricht dann der Überlagerung der relativen Lage der Bezugssysteme bei der Aufnahme des dreidimensionalen Datensatzes mit den Lageänderungsdaten.

Optional wird aus der relativen Lage der Wirbel eine dreidimensionale Darstellung des aktuellen Zustands der Wirbelsäule analog zu der Darstellung aus Figur 3 erzeugt. Diese Darstellung dient dem Operateur als visuelle Rückmeldung über die aktuelle relative Lage der Wirbel.

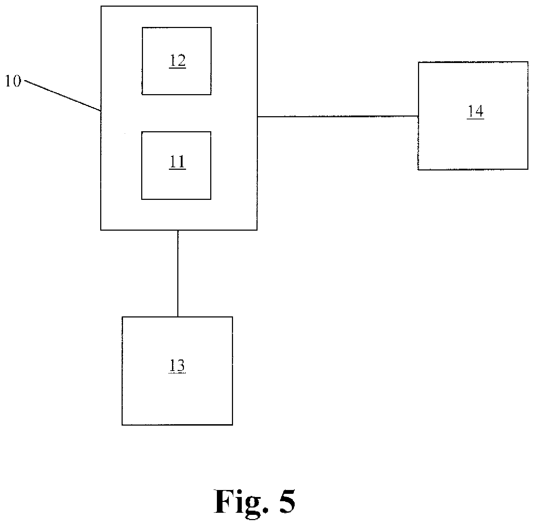

Die Figur 5 zeigt schematisch eine Vorrichtung zur Bestimmung der relativen Lage von Knochenstrukturen. Die Vorrichtung weist einen Computer 10 auf, der unter anderem einen Speicher 11 und eine Recheneinheit 12 enthält. Mit dem Computer 10 verbunden sind ein Computertomograph 13 und ein Röntgenapparat 14. Der Computertomograph 13 generiert den dreidimensionalen Datensatz, der im Speicher 11 des Computers 10 hinterlegt wird. Dazu kann eine direkte Verbindung zwischen dem Computertomographen 13 und dem Computer 10 bestehen. Alternativ werden die Daten über ein Netzwerk oder mithilfe eines Datenträgers vom Computertomographen 13 in den Speicher 11 übertragen. Die Segmentierung des dreidimensionalen Datensatzes erfolgt im Computertomographen 13 oder im Computer 10.

Der Röntgenapparat 14 erzeugt Röntgenbilder, die Informationen über die Lage der Knochenstrukturen enthalten. Diese Röntgenbilder werden an den Computer 10 übertragen und dort von der Recheneinheit 12 ausgewertet. Die Recheneinheit 12 berechnet die Lageänderungsdaten der Segmente sowie die relative Lage der Knochenstrukturen und vergleicht diese mit einer Solllage. Eine Ausgabeeinrichtung, beispielsweise ein Monitor, zur Wiedergabe einer Darstellung der Knochenstrukturen in ihrer relativen Lage zum Zeitpunkt der Aufnahme der Röntgenbilder ist in der Figur 5 nicht dargestellt.

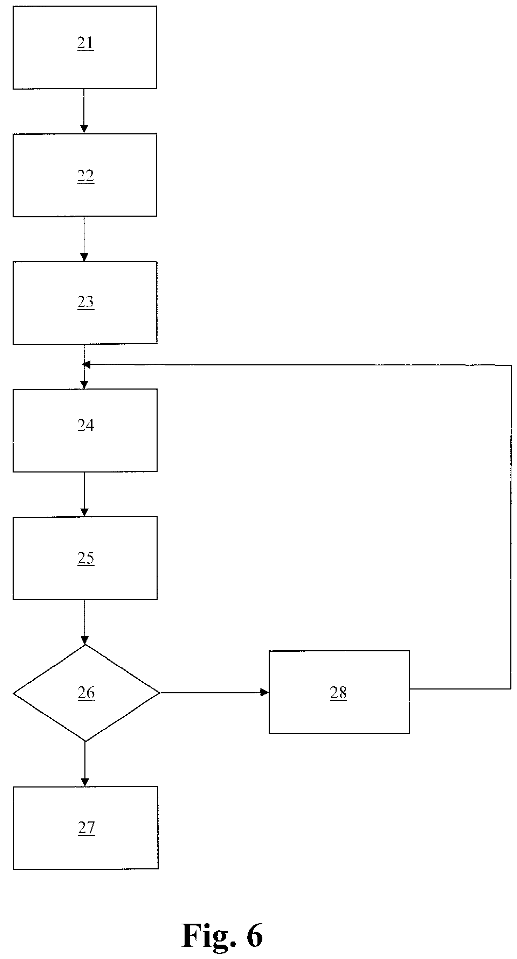

Die Figur 6 zeigt ein Flussdiagramm eines Verfahrens zur Überprüfung der relativen Lage von Knochenstrukturen. Dabei wird in einem ersten Schritt 21 ein präopcrativer Scan der Knochenstrukturen durchgeführt und dabei ein dreidimensionaler Datensatz erzeugt. Im Schritt 22 erfolgt eine Segmentierung des dreidimensionalen Datensatzes in Segmente, die jeweils eine Knochenstruktur repräsentieren. Im Schritt 23 wird jedem der Segmente ein individuelles Bezugssystem zugeordnet. Im Schritt 24 erfolgt eine intraoperative Bestimmung der Lagen der Knochenstrukturen mithilfe des CT-Fluoro-Matching wie vorstehend beschrieben. Das Resultat sind Lageänderungsdaten, aus denen unter Berücksichtigung der Lage der Bezugssysteme im dreidimensionalen Datensatz die relative Lage der Bezugssysteme zum Zeitpunkt der Aufnahme der Röntgenbilder berechnet wird. Die Lagen der Knochenstrukturen können im Schritt 24 ergänzend oder alternativ mittels so genannter Pointer bestimmt werden. Die Pointer umfassen detektierbare Marker, deren Lage zu Pointerspitze bekannt sind. Die Pointerspitze wird insbesondere mit Landmarken der Knochenstrukturen in Kontakt gebracht.

Im Schritt 25 wird aus den Segmenten und ihrer virtuellen Lage eine Ansicht der Knochenstrukturen in ihrer relativen Lage dargestellt.

Im Schritt 26 wird überprüft, ob die relative Lage der Bezugssysteme zueinander einer festgelegten Solllage entspricht, wobei optional eine Toleranz zulässig ist. Entspricht die relative Lage der Solllage, so wird im Schritt 27 eine entsprechende Meldung ausgegeben.

Entspricht die relative Lage nicht der Solllage, so wird im Schritt 28 die Position des Patienten und somit die relative Lage der Knochenstrukturen verändert. Anschließend wird das Verfahren mit Schritt 24 fortgesetzt.

Optional sind eine oder mehrere der Knochenstrukturen mit Markiervorrichtungen versehen, beispielsweise mit Markersternen. Ein Markerstern ist eine dreidimensionale Anordnung von drei oder mehr Kugeln, deren Lage mithilfe einer Erfassungsvorrichtung wie einer 3D-Kamera ermittelt werden kann. Aufgrund der festen Verbindung zwischen der Markiervorrichtung und der markierten Struktur kann so auf die Lage der Struktur geschlossen werden. Mithilfe der Markervorrichtung ist eine Bestimmung der Lage der markierten Knochenstruktur möglich. Insbesondere können Hinweisinformationen (z.B. Anzeige an Bildschirm, Audio etc.) ausgegeben werden, die über die Lage informieren. Wird auch die Lage der mit Markiervorrichtungen versehenen Knochenstrukturen anhand des vorstehend beschriebenen Verfahrens berechnet, so können die Markiervorrichtungen zur Kontrolle des Verfahrens verwendet werden, indem die gemessene Lage der markierten Knochenstrukturen mit der berechneten Lage verglichen wird.

Der dreidimensionale Datensatz kann anstatt mittels einer Computertomographie auch mit jedem anderen dreidimensional bildgebenden Verfahren aufgenommen werden, beispielsweise unter Verwendung eines Magnetresonanztomographen.

## Patentansprüche

1. Verfahren zur Überprüfung der relativen Lage mindestens zweier Knochenstrukturen (1, 2), insbesondere mindestens zweier Wirbel einer Wirbelsäule, zueinander, wobei der Begriff "Lage" sowohl die Position als auch eine Orientierung umfasst, aufweisend die Verfahrensschritte
- Aufnehmen eines dreidimensionalen Bilddatensatzes, der die Knochenstrukturen (1,2) darstellt, zu einem ersten Zeitpunkt
- Segmentieren des dreidimensionalen Bilddatensatzes in Segmente, wobei jedes Segment eine Knochenstruktur (1,2) darstellt und die Segmente im dreidimensionalen Bilddatensatz eine bekannte virtuelle relative Lage aufweisen
- Ändern der virtuellen relativen Lage der Segmente, sodass die virtuelle relative Lage der Segmente mit der realen relativen Lage der Knochenstrukturen (1, 2) zu einem späteren Zeitpunkt übereinstimmt
- Überprüfen, ob die virtuelle relative Lage der Segmente mit einer Solllage der Knochenstrukturen (1, 2) übereinstimmt.

2. Verfahren nach Anspruch 1, **dadurch gekennzeichnet, dass** die Änderung der virtuellen relativen Lage der Segmente anhand von CT-Fluoro-Matching erfolgt.

3. Verfahren nach Anspruch 1, **dadurch gekennzeichnet, dass** die Änderung der virtuellen relativen Lage der Segmente dadurch erfolgt, dass Landmarken der Knochenstrukturen (1, 2) mittels eines Pointers abgetastet werden und die virtuelle relative Lage der Segmente geändert wird, bis korrespondierende Landmarken der Segmente mit den abgetasteten Landmarken übereinstimmen.

4. Verfahren nach einem der Ansprüche 1 bis 3, **dadurch gekennzeichnet, dass** das Aufnehmen des dreidimensionalen Bilddatensatzes präoperativ erfolgt.

5. Verfahren nach einem der Ansprüche 1 bis 4, **dadurch gekennzeichnet, dass** die Überprüfung, ob die virtuelle relative Lage der Segmente mit einer Solllage der Knochenstrukturen (1, 2) übereinstimmt, dadurch erfolgt, dass jedem Segment des dreidimensionalen Bilddatensatzes ein individuelles Bezugssystem zugeordnet wird und die relative Lage der Bezugssysteme mit einer Solllage der Bezugssysteme verglichen wird, die zu der Solllage der Knochenstrukturen (1, 2) korrespondiert.

6. Verfahren nach einem der Ansprüche 1 bis 5, **dadurch gekennzeichnet, dass** das Bezugssystem aus einer Sagittalebene und einer Koronarebene besteht.

7. Verfahren nach einem der Ansprüche 1 bis 6, **dadurch gekennzeichnet, dass** die Solllage der Knochenstrukturen (1, 2) durch Festlegen eines Lordosewinkels beziehungsweise Kyphosewinkels und/oder einer Translation in medio-lateraler und/oder anterior-posteriorer Richtung definiert wird.

8. Verfahren nach einem der Ansprüche 1 bis 7, **dadurch gekennzeichnet, dass** bei der Überprüfung, ob die relative Lage der Knochenstrukturen (1, 2) der Solllage der Knochenstrukturen entspricht, eine vorgebbare Abweichung zulässig ist.

9. Verfahren nach einem der Ansprüche 1 bis 8, **dadurch gekennzeichnet, dass** aus den Segmenten und ihrer virtuellen relativen Lage eine Ansicht der Knochenstrukturen (1, 2) in ihrer relativen Lage berechnet und dargestellt wird.

10. Programm, das, wenn es in eine Datenverarbeitungseinrichtung (10) geladen wird oder auf einer Datenverarbeitungseinrichtung (10) läuft, die Datenverarbeitungseinrichtung (10) veranlasst, das Verfahren nach einem der Ansprüche 1 bis 10 auszuführen.

11. Speichermedium, auf dem das Programm nach Anspruch 10 gespeichert ist, oder Signalwelle, die Informationen trägt, die das Programm nach Anspruch 10 darstellen.

12. Vorrichtung, aufweisend einen Computer (10), auf dem das Programm nach Anspruch 10 läuft oder geladen ist.

## Claims

1. Method for checking the relative location of at least two bone structures (1, 2), in particular at least two vertebrae of a spinal column, with respect to one another, wherein the term "location" comprises both the position and orientation, having the method steps
- receiving a three-dimensional image data set which represents the bone structures (1, 2), at a first point in time
- segmenting the three-dimensional image data set into segments, wherein each segment represents a bone structure (1, 2) and the segments in the three-dimensional image data set have a known virtual relative location
- changing the virtual relative location of the segments such that the virtual relative location of the segments corresponds to the actual relative location of the bone structures (1, 2) at a later point in time
- checking whether the virtual relative location of the segments corresponds to a desired location of the bone structures (1,2).

2. Method according to claim 1, **characterised in that** the virtual relative location of the segments is changed by means of CT-fluoro matching.

3. Method according to claim 1, **characterised in that** the virtual relative location of the segments is changed by landmarks of the bone structures (1, 2) being scanned by means of a pointer and the virtual relative location of the segments being changed until corresponding landmarks of the segments correspond to the scanned landmarks.

4. Method according to one of claims 1 to 3, **characterised in that** the three-dimensional image data set is received pre-operatively.

5. Method according to one of claims 1 to 4, **characterised in that** checking whether the virtual location of the segments corresponds to a desired position of the bone structures (1, 2) takes place by an individual reference system being allocated to each segment of the three-dimensional image data set and the relative location of the reference system being compared to a desired location of the reference system, which corresponds to the desired location of the bone structures (1,2).

6. Method according to one of claims 1 to 5, **characterised in that** the reference system consists of a sagittal plane and a coronal plane.

7. Method according to one of claims 1 to 6, **characterised in that** the desired location of the bone structures (1, 2) is defined by determining a lordosis angle or kyphosis angle and/or a translation in the medio-lateral and/or anterior-posterior direction.

8. Method according to one of claims 1 to 7, **characterised in that** when checking whether the relative location of the bone structures (1, 2) corresponds to the desired location of the bone structures, a predeterminable deviation is permitted.

9. Method according to one of claims 1 to 8, **characterised in that** a view of the bone structures (1, 2) in their relative location is calculated and shown from the segments and their virtual relative location.

10. Program which, when it is uploaded to a data processing unit (10) or runs on a data processing unit (10), prompts the data processing unit (10) to carry out the method according to one of claims 1 to 10.

11. Storage medium on which the program according to claim 10 is saved, or signal wave which carries information which shows the program according to claim 10.

12. Device having a computer (10) on which the program runs or onto which the program is uploaded according to claim 10.

## Revendications

1. Procédé permettant de vérifier la situation relative d'au moins deux structures osseuses (1, 2), en particulier d'au moins deux vertèbres d'une colonne vertébrale, entre elles, le terme de « situation » recouvrant aussi bien la position qu'une orientation, comprenant les étapes de procédé suivantes :
- l'enregistrement d'un ensemble de données d'images en trois dimensions représentant les structures osseuses (1, 2), à un premier instant,
- la segmentation de l'ensemble de données d'images en trois dimensions en segments, chaque segment représentant une structure osseuse (1, 2), lesdits segments de l'ensemble de données d'images en trois dimensions présentant une situation relative virtuelle connue,
- la modification de la situation relative virtuelle des segments, de manière que la situation relative virtuelle des segments coïncide avec la situation relative réelle des structures osseuses (1, 2) à un instant ultérieur,
- la vérification que la situation relative virtuelle des segments coïncide avec une position de consigne des structures osseuses (1, 2).

2. Procédé selon la revendication 1, **caractérisé en ce que** la modification de la situation relative virtuelle des segments s'effectue par fluoro-TDM.

3. Procédé selon la revendication 1, **caractérisé en ce que** la modification de la situation relative virtuelle des segments s'effectue en détectant des points de repère des structures osseuses (1, 2) au moyen d'un pointeur et la situation relative virtuelle des segments est modifiée jusqu'à ce que des points de repère correspondants des segments coïncident avec les points de repère détectés.

4. Procédé selon l'une des revendications 1 à 3, **caractérisé en ce que** l'enregistrement de l'ensemble de données d'images en trois dimensions s'effectue de manière préopératoire.

5. Procédé selon l'une des revendications 1 à 4, **caractérisé en ce que** la vérification que la situation relative virtuelle des segments coïncide avec une position de consigne des structures osseuses (1, 2) s'effectue en associant un système de référence individuel à chaque segment de l'ensemble de données d'images en trois dimensions et en comparant la situation relative des systèmes de référence à une position de consigne des systèmes de référence qui correspond à la position de consigne des structures osseuses (1, 2).

6. Procédé selon l'une des revendications 1 à 5, **caractérisé en ce que** le système de référence se compose d'un plan sagittal et d'un plan coronaire.

7. Procédé selon l'une des revendications 1 à 6, **caractérisé en ce que** la position de consigne des structures osseuses (1, 2) est définie par la détermination d'un angle lordotique ou cyphotique et/ou d'une translation dans une direction médio-latérale et/ou antérieure-postérieure.

8. Procédé selon l'une des revendications 1 à 7, **caractérisé en ce que**, lors de la vérification que la position relative des structures osseuses (1, 2) correspond à la position de consigne des structures osseuses, un écart spécifié est autorisé.

9. Procédé selon l'une des revendications 1 à 8, **caractérisé en ce que**, à partir des segments et de leur situation relative virtuelle, une vue des structures osseuses (1, 2) dans leur situation relative est calculée et représentée.

10. Programme qui, lorsqu'il est chargé dans un dispositif de traitement de données (10) ou est exécuté sur un dispositif de traitement de données (10), amène le dispositif de traitement de données (10) à exécuter le procédé selon l'une des revendications 1 à 10.

11. Support d'enregistrement sur lequel est enregistré le programme selon la revendication 10, ou onde de signal porteuse d'informations représentant le programme selon la revendication 10.

12. Dispositif comprenant un ordinateur (10) sur lequel le programme selon la revendication 10 est exécuté ou chargé.
